# EUROPÄISCHE PATENTSCHRIFT

(11) **EP 3 887 719 B1**
(45) Veröffentlichungstag und Bekanntmachung des Hinweises auf die Patenterteilung: **26.06.2024**
(21) Anmeldenummer: 19780177.2
(22) Anmeldetag: 21.09.2019
(51) Int. Cl.: F23N 5/24, G01M 3/20, G01M 3/38, G01N 21/35, G01N 33/00

(54) **VERFAHREN ZUM DETEKTIEREN EINES VERBRENNUNGSGASES**
METHOD FOR DETECTING A COMBUSTION GAS
PROCÉDÉ POUR DÉTECTER UN GAZ DE COMBUSTION

(30) Priorität: 29.11.2018 DE 102018220610
(43) Veröffentlichungstag der Anmeldung: 06.10.2021
(73) Patentinhaber: Robert Bosch GmbH, 70442 Stuttgart (DE)
(72) Erfinder: HULSHOF, Dik Jan, 7274 Geesteren (NL); SMITH, Lean, 8171 KC Vaassen (NL)
(86) Internationale Anmeldenummer: PCT/EP2019/075435
(87) Internationale Veröffentlichungsnummer: WO 2020/108819

(56) Entgegenhaltungen:
- EP-A1- 2 119 965
- US-A- 4 560 873
- US-A- 4 810 884
- US-B1- 8 947 249

## Beschreibung

Die Erfindung betrifft ein Verfahren zum Detektieren von Verbrennungsgas eines Heizelements durch ein Erfassungsmittel in einem durch ein Gehäuse einer Heizvorrichtung gebildeten Innenraum nach dem unabhängigen Anspruch.

Es sind Heizvorrichtungen mit Heizelementen bekannt, in denen kohlenstoffhaltige Brennstoffe verbrannt werden. Bei dieser Verbrennung entstehen Verbrennungsgase wie CO2, die durch ein Abgasrohr aus der Heizvorrichtung entfernt werden. Allerdings kann das Heizelement undicht sein, sodass Verbrennungsgase in den Innenraum der Heizvorrichtung treten. Bleibt dies für längere Zeit unerkannt, können beispielsweise durch Überhitzung Schäden an der Heizvorrichtung und dem Heizelement auftreten. Ferner verringert sich die Effizienz der Heizvorrichtung bei undichten Heizelementen drastisch.

Ausgehend hiervon liegt der Erfindung die Aufgabe zugrunde, die im Stand der Technik bekannten Heizvorrichtungen für elektrische Maschinen weiter zu verbessern und ein Verfahren für eine Heizvorrichtung bereitzustellen, das eine Leckage in einem Heizelement erkennt. Verfahren des Standes der Technik sind in den Dokumenten US 4560873A , US 8947249 B1 und EP 2119965 A1 offenbart.

Zur Lösung dieser Aufgabe wird die in dem unabhängigen Patentanspruch angegebene Merkmalskombination vorgeschlagen. Vorteilhafte Ausgestaltungen und Weiterbildungen der Erfindung ergeben sich aus den abhängigen Ansprüchen.

Es ist darauf hinzuweisen, dass die in der nachfolgenden Beschreibung einzeln aufgeführten Merkmale sowie Maßnahmen in beliebiger, technisch sinnvoller Weise miteinander kombiniert werden können und weitere Ausgestaltungen der Erfindung aufzeigen. Die Beschreibung charakterisiert und spezifiziert die Erfindung insbesondere im Zusammenhang mit den Figuren zusätzlich.

Das erfindungsgemäße Erfassungsmittel für ein Verbrennungsgas in einer Heizvorrichtung umfasst eine Umhausung in der eine Spektroskopieeinheit mit einem Sensor zur Erfassung des Verbrennungsgases und einer Erfassungsmittelsteuerung positioniert ist, wobei die Umhausung wenigstens eine Öffnung für den Eintritt des Verbrennungsgases aufweist. Die Öffnung kann z.B. einen Querschnitt von in etwa 19 mm² aufweisen. Beispielsweise kann die Öffnung durch ein kreisrundes Loch mit einem Durchmesser 5 mm bereitgestellt werden. Ferner erwärmt die Heizvorrichtung mit dem eine Verbrennung beherbergenden Heizelement ein in thermischen Kontakt stehenden Mediums durch die Abwärme der Verbrennung, wobei ein Verbrennungsgas wie CO2 ein Produkt der Verbrennung ist, und ein das Heizelement umhausendes und einen Innenraum bildendes Gehäuse.

Das Erfassungsmittel führt das erfindungsgemäße Verfahren zum Detektieren von Verbrennungsgas aus, wobei das Erkennen durch eine spektroskopische Messmethode mittels einer Spektroskopieeinheit und einen Sensor innerhalb einer Umhausung des Erfassungsmittels erfolgt, sodass bei Überschreiten eines bestimmten Schellenwerts der Konzentration des Verbrennungsgases im Innenraum durch eine Erfassungsmittelsteuerung ein Warnsignal von dem Erfassungsmittel ausgegeben wird. Beim Überschreiten eines oberen Schwellenwerts der Konzentration erkennt die Erfassungsmittelsteuerung einen zu hohen Gehalt an Verbrennungsgas in der Luft des Innenraums des Gehäuses und es kann anhand der erhöhten Konzentration ein Defekt abgeleitet werden, der dazu führt, das Verbrennungsgas nicht durch ein Abgasrohr aus der Heizvorrichtung geleitet wird, sondern in den Innenraum des Gehäuses entweicht. Solch ein Verfahren ist besonders vorteilhaft, wenn man eine sichere Heizvorrichtung bereitstellen möchte, da beispielsweise ein Leck in dem Heizelement frühzeitig erkannt werden kann und übliche Folgeschäden vermieden werden können. Der Schwellenwert der Konzentration kann in der Erfassungsmittelsteuerung hinterlegt sein.

Ferner kann eine geregelte Stilllegung der Heizvorrichtung gesteuert werden, indem eine charakteristische Erhöhung der Konzentration an Verbrennungsgas in dem Innenraum des Gehäuses über einem bestimmten Zeitraum erkannt wird. Somit kann eine hohe Sicherheit und Effizienz bei der Anwendung der Heizvorrichtung erreicht werden.

Das erfindungsgemäße Erfassungsmitel detektiert die Konzentration des Verbrennungsgases in der Luft des Innenraums, wobei der obere Schwellenwert der Konzentration in etwa zwischen 2000 und 1000 ppm und insbesondere bei 7000 ppm liegt, wobei der obere Schwellenwert vorzugsweise in einem Speicher in der Erfassungsmittelsteuerung hinterlegt ist. Dabei erkennt der Sensor mithilfe der Spektroskopieeinheit die Höhe der Konzentration, wobei der Sensor von der Erfassungsmitteleinheit ausgelesen wird und der ausgelesene Wert wird mit dem im Speicher hinterlegten Wert verglichen.

Damit sichere eine Ausgabe des Warnsignals erfolgt, sollte die bestimmte Konzentration eine bestimmte Zeitspanne lang durch das Erfassungsmittel erkannt werden. Die bestimmte Zeitspanne ist ein fester Zeitwert, der in der Erfassungsmittelsteuerung hinterlegt ist. Der Zeitwert kann je nach Umweltbedingungen wie Temperatur, Feuchtigkeit, Alter der Heizvorrichtung und/oder Luftdruck variiert werden und auf einen weiteren festen Zeitwert festgelegt werden. Die Zeitspanne kann zwischen wenigen Sekunden bis zu Jahren liegen. Beispielsweise kann die Zeitspanne 15 s, 30 s, 5 min und/oder 48 h betragen.

Es kann bei einer vorteilhaften Weiterbildung vorgesehen werden, dass eine Häufigkeit der Überschreitung des oberen Schwellenwerts durch die Erfassungsmittelsteuerung insbesondere während einer bestimmten Zeitspanne gezählt wird, und das Warnsignal erst ausgegeben wird, wenn die Häufigkeit einen bestimmten Häufigkeitswert überschreitet. Jede Überschreitung kann als ein Ereignis gezählt werden, wobei der bestimmte Häufigkeitswert in der Erfassungsmittelsteuerung hinterlegt sein kann. Das Warnsignal kann mit dem Erreichen des bestimmten Häufigkeitswerts oder mit einem weiteren Ereignis nach dem Erreichen des bestimmten Häufigkeitswerts ausgegeben werden. Der bestimmte Häufigkeitswert kann zum Beispiel 10, 20 und/oder 40 Ereignisse betragen. Ferner kann der Häufigkeitswert aufgrund veränderter Umweltparameter variieren.

Um eine sichere Betriebsweise der Heizvorrichtung zu gewährleisten kann vorzugsweise vor dem insbesondere ersten Messen der Konzentration des Verbrennungsgases durch die Erfassungsmittelsteuerung geprüft werden, ob der Sensor eine Konzentration erkennt, die unter oder gleich einem bestimmten unteren Schwellenwert liegt, wobei der untere Schwellenwert insbesondere in etwa zwischen 0 und 400 ppm liegt. Die durchschnittliche CO2-Konzentration in der Luft liegt bei ca. 400 ppm. Falls der Sensor einen Wert unterhalb der durchschnittlichen Konzentration oder gar unter 0 ppm feststellt, dann ist von einem Fehler an dem Erfassungsmittel auszugehen. Der Fehler kann an dem den Wert der Konzentration bestimmenden Sensor vorliegen. Wenn die Konzentration bei der Messung unter dem unteren Schwellenwert liegt oder gleich dem unteren Schwellenwert ist, wird von der Erfassungsmittelsteuerung ein Fehlersignal ausgegeben, das einen Fehler des Sensors und/oder der Spektroskopieeinheit bekundet.

Ferner kann der Häufigkeitswert aufgrund veränderter Umweltparameter variieren. Als Ereignis einer Häufigkeitszählung kann auch eine Häufigkeit der Unterschreitung des unteren Schwellenwerts durch die Erfassungsmittelsteuerung insbesondre während einer bestimmten Zeitspanne gezählt werden. Das Fehlersignal wird erst ausgegeben, wenn die Häufigkeit einen bestimmten Häufigkeitswert überschreitet. Hierbei kann der bestimmte Häufigkeitswert zum Beispiel 10, 20 und/oder 40 Ereignisse betragen. Das Fehlersignal kann mit dem Erreichen des bestimmten Häufigkeitswerts oder mit einem weiteren Ereignis nach dem Erreichen des bestimmten Häufigkeitswerts ausgegeben werden.

Bevorzugterweise kann das Warnsignal und/oder das Fehlersignal an eine Steuerung der Heizvorrichtung und/oder an eine Anzeige des Erfassungsmittels abgegeben werden. Dadurch kann die Heizvorrichtung und/oder die Anzeige in einen Zustand versetzt werden, der zur Beseitigung des Warnsignals und/oder des Fehlersignals gereicht.

Die Steuerung der Heizvorrichtung kann eine Verringerung der Konzentration des Verbrennungsgases bewirken, wenn zum Beispiel das Warnsignal ein Ausschalten der Heizvorrichtung auslöst, sodass die Verbrennung erlischt. Mit Erlöschen der Verbrennung wird kein Verbrennungsgas mehr erzeugt, sodass dessen Konzentration im Innenraum abfallen kann. Alternativ oder ergänzend kann ein Blockieren der Steuerung der Heizvorrichtung ausgelöst werden, sodass entweder ein Verstärken der Verbrennung, wenn die Verbrennung gedrosselt oder konstant gehalten wird, nicht möglich ist. Ferner ist nach dem Blockieren der Steuerung ein Wiedereinschalten der Verbrennung nicht mehr möglich, falls die Verbrennung gelöscht wurde. Weiter alternativ oder ergänzend kann eine Verringerung der Verbrennung innerhalb des Heizelements bewirkt werden, sodass die Verbrennung beispielsweise in einem Notmodus auf geringer Leistung gehalten wird und dabei wenig Verbrennungsgas produziert.

Es kann von Vorteil sein, die Heizvorrichtung nach dem Ausgeben des Warnsignals und/oder des Fehlersignals durch einen Fortführbefehl weiterzubetreiben. Die Erfassungsmittelsteuerung kann hierbei die Häufigkeit der Fortführbefehle insbesondere während einer bestimmten Zeitspanne zählen. Die bestimmte Zeitspanne kann in der Erfassungsmittelsteuerung hinterlegt werden. Bei Überschreiten einer bestimmten Häufigkeit kann die Heizvorrichtung ausgeschaltet werden und/oder die Steuerung der Heizvorrichtung blockiert werden und/oder die Verbrennung verringert werden. Alternativ oder ergänzend kann der Fortführbefehl blockiert werden, wodurch die Heizvorrichtung stillgelegt wird, wenn die Konzentration erneut den oberen Schwellenwert überschreitet. Ferner kann die Konzentration des Verbrennungsgases durch die Blockade des Fortführbefehls reduziert werden.

Zweckmäßigerweise kann eine Anzeige, die das Warnsignal und/oder das Fehlersignal empfängt, an der Umhausung angeordnet sein, wobei die Anzeige wenigstens eine LED ist, die je nach Messergebnis des Sensors blinkt oder ausgeschaltet ist oder durchgehend leuchtet, wobei die LED insbesondere in unterschiedlichen Farben leuchten kann. Beispielsweise kann die Anzeige durchgehend leuchten, wenn der obere Schwellenwert überschritten wird.

Für eine optimale Steuerbarkeit des Erfassungsmittels kann die Erfassungsmittelsteuerung über eine Antenne Funksignale an ein bezüglich des Erfassungsmittels externes Gerät wie einem Computer, einem Smart Phone und/oder ein Tablet sendet, und einen Zustand der Heizvorrichtung und/oder eine Konzentration des Verbrennungsgases und/oder ausgegebene Signale und/oder Häufigkeiten auf dem jeweiligen externen Gerät anzeigen. Hierzu kann beispielsweise Bluetooth, Wlan, Zigbee oder ein anderer Funkstandard verwendet werden.

Eine vorteilhafte Ergänzung kann durch die Antenne erreicht werden, wenn Funksignale empfangbar sind, sodass durch ein externes Gerät wie einem Computer, einem Smart Phone und/oder ein Tablet das Erfassungsmittel steuerbar ist. Dadurch kann über eine App auf dem externen Gerät das Erfassungsmittel so gesteuert werden, dass beispielsweise Fortführbefehle und/oder Blockaden eingeschaltet und/oder aufgehoben werden. Ferner kann die Heizvorrichtung stillgelegt werden und/oder der Verbrennung reduziert und/oder verstärkt und/oder konstant gehalten werden.

Die Konzentration des Verbrennungsgases kann von dem Luftdruck, der Luftfeuchtigkeit und der Temperatur abhängen. Diese Größen verschieben zum Beispiel den Wert der durchschnittlichen Konzentration von CO2 in der Luft. Ferner kann aufgrund starker Verbrennung im Heizelement auch im Normalbetrieb ein erhöhter Wert in dem Innenraum des Gehäuses vorliegen. Ein Indikator für eine starke Verbrennung ist eine erhöhte Drehzahl des Gebläses, da der Verbrennung mehr Luft zur Verfügung gestellt werden muss, um das Verbrennungsgas und die damit verbünde Abwärme zu produzieren. Auch eine erhöhte Luftfeuchtigkeit kann ein Hinweis auf eine stärkere Verbrennung sein, da H2O bei der Verbrennung von kohlenstoffhaltigen Stoffen wie Erdgas entsteht und in den Innenraum gelangt. Damit diese Verschiebung berücksichtigt wird, kann wenigstens ein Signal eines Drehzahlmessers eines Gebläses, Feuchtigkeitssensors, Drucksensor, der Heizvorrichtung und/oder eines Temperatursensors von der Erfassungsmittelsteuerung herangezogen werden, um den oberen Schwellenwert der Konzentration entsprechend anzupassen. Dadurch kann beispielsweise eine Heizvorrichtung die in Regionen mit geringerem Luftdruck als der Normaldruck und hoher Luftfeuchtigkeit optimal überwacht werden.

Um eine verlässliche Überwachung des Gebläses zu gewährleisten, kann die Drehzahl eines Gebläses abgeprüft werden, und wenn die Drehzahl des Gebläses niedriger als ein Sollwert ist, wird eine Anzeige wie eine LED des Erfassungsmittels insbesondere in einen blickenden Zustand eingeschaltet.

Vorteilhafterweise können Kontakte für eine Datenübertragung insbesondere auf einer Unterseite und/oder einer Seitenfläche der Umhausung des Erfassungsmittels angeordnet sein. Die Kontakte können unmittelbar an der Oberfläche positioniert sein. Durch die Kontakte können Daten, die in einem Speicher des Erfassungsmittels aus vergangenen Messungen hinterlegt sind, herausgelesen werden. Die Daten können beispielsweise Fehlermeldungen, Konzentrationsabgeben des Verbrennungsgases, Häufigkeit der Überschreitung des Schwellenwerts, zeitlicheTemperatur- und Luftdruckverläufe und/oder Zeitpunkte und Zeitdauern von Ereignissen wie Überschreitung des Schwellenwerts umfassen. Zum Auslesen des Speichers kann ein Lesegerät mit den Kontakten verbunden werden.

Die Heizvorrichtung mit dem eine Verbrennung beherbergenden Heizelement erwärmt durch die Abwärme der Verbrennung ein mit dem Heizelement in thermischen Kontakt stehendes Medium. Das Verbrennungsgas, z.B. CO2, entsteht als ein Produkt der Verbrennung. Die Heizvorrichtung kann durch das Erfassungsmittel für das Verbrennungsgas in einer Heizvorrichtung erfindungsgemäß geschützt werden. Dazu wird das Erfassungsmittel innerhalb eines Gehäuses der Heizvorrichtung positioniert, sodass Verbrennungsgas, welches aus einem Heizelement der Heizvorrichtung austritt und sich innerhalb eines Innerraums des Gehäuses ansammelt, erkannt wird. Dazu ist das Erfassungsmittel mit einer Umhausung, in der eine Spektroskopieeinheit mit einem Sensor zur Erfassung des Verbrennungsgases und eine Erfassungsmittelsteuerung positioniert ist, ausgeführt. Die Umhausung weist Öffnungen für den Eintritt des Gemisches aus Verbrennungsgas und Luft aus dem Innenraum des Gehäuses der Heizvorrichtung auf. Das in die Umhausung eingetretene Verbrennungsgas wird durch die Spektroskopieeinheit analysiert und durch den Sensor wird eine bestimmte Konzentration an Verbrennungsgas eines bestimmten Typs wie CO2 erkannt. Die Konzentration kann beispielsweise zwischen 2000 bis 10 000 ppm (parts per million) betragen. Damit in das Erfassungsmittel kein Schmutz wie Staub durch die Öffnungen eintritt, ist die Öffnung in Erdanziehungsrichtung nach unten gerichtet. Ferner ist der Sensor ebenfalls nach unten gerichtet, sodass sich kein Schmutz oder eintretende Flüssigkeit auf dem Sensor ablagern kann.

Um den Zustand einer Heizvorrichtung und/oder des Erfassungsmittels schnell erkennen zu können, ist es besonders vorteilhaft eine visuelle Anzeige an der Umhausung des Erfassungsmittels vorzusehen. Die Anzeige kann vorzugsweise als LED ausgebildet sein, die je nach Zustand in unterschiedlichen Farben und Frequenzen blinkt oder durchgehend leuchtet oder ausgeschaltet ist. Ferner kann auch eine akustische Anzeige erfolgen, sodass ein Piepton bei bestimmten Zuständen ausgegeben wird.

Damit das Erfassungsmittel schnell in dem Innenraum des Gehäuses der Heizvorrichtung fixiert werden kann, kann wenigstens ein Befestigungsmittel an der Umhausung angeformt werden. Das Befestigungsmittel kann insbesondere als Schelle ausgebildet sein. Das Befestigungsmittel kann an einem weiteren Bauteil innerhalb der Heizvorrichtung mechanisch angebracht werden, sodass eine definierte starre Ausrichtung des Erfassungsmittels erfolgt.

Vorzugsweise können wenigstens zwei Befestigungsmittel auf der Unterseite angebracht werden, und insbesondere in unterschiedliche um 90° zueinander versetzte Vorzugsrichtungen ausgerichtet sein. So kann das Erfassungsmittel gegen ein Verdrehen gegenüber der horizontalen in zwei Richtungen gesichert werden, wobei es beispielsweise auf einem gebogenen Rohr befestigt werden kann. Das Erfassungsmittel könnte auf ein Ansaugrohr eines Gebläses der Heizvorrichtung befestigt werden, durch welches Luft für die Verbrennung in das Heizelement angesaugt wird.

Zweckmäßigerweise kann das Erfassungsmittel an eine Spannungsversorgung eines in dem Innenraum des Gehäuses angeordneten Gebläses zur Versorgung der Verbrennung mit Luft angeschlossen werden. Dabei kann das Erfassungsmittel vorzugsweise zu weiteren Bauteilen der Heizvorrichtung parallelgeschaltet sein. Somit kann eine Synchronisation des Gebläses und des Erfassungsmittels erreicht werden, bei der ein Einschalten des Gebläses und/oder der gesamten Heizvorrichtung ein Starten des Erfassungsmittels bewirkt.

Insbesondere für CO2-Messungen ist es von Vorteil, wenn das Erfassungsmittel eine auf infrarotem Licht basierende Spektroskopieeinheit enthält, die von einer Umhausung umgeben ist, die wenigstens eine Öffnung für den Eintritt des Verbrennungsgases aufweist. In der Erfassungseinheit wird das CO2 enthaltende Gemisch aus Verbrennungsgas und Luft des Innenraums der Heizvorrichtung durch die Spektroskopieeinheit analysiert und eine Konzentration an CO2 gemessen.

Besonders bevorzugt ist eine Ausführungsform, bei der in dem Erfassungsmittel mindestens zwei Sensoren und/oder Spektroskopieeinheiten enthalten sind, die die gleiche Messung ausführen. Dadurch wird eine redundante Messung ausgeführt, wobei die einzelnen Messungen durch die Erfassungsmittelsteuerung miteinander verglichen werden, um eine möglichst hohe Sicherheit hinsichtlich der Konzentration an Verbrennungsgas im Innenraum der Heizvorrichtung zu erhalten.

Bei einer weiteren Ausführungsform kann vorgesehen werden, dass das Erfassungsmittel in einem bezüglich der Erdanziehungsrichtung unteren Teil des Innenraums angeordnet ist. Da insbesondere CO2 schwerer ist als Luft, kann das aus der Verbrennung und einer Leckage des Heizelements stammende CO2 in den unteren Teil des Innenraums in den Erfassungsbereich des Erfassungsmittels absinken.

Vorteilhafte Ausführungsbeispiele der Erfindung sind schematisch in den Figuren dargestellt und werden nachfolgend beschrieben. In den unterschiedlichen Figuren sind gleiche Teile stets mit denselben Bezugszeichen versehen, weswegen diese in der Regel auch nur einmal beschrieben werden.

Es zeigen
- Fig. 1: A und B eine Unteransicht bzw. eine Seitenansicht eines Erfassungsmittels zum Erkennen eines Verbrennungsgases,
- Fig. 2: A und B eine schematische Darstellung einer Heizvorrichtung mit einem Heizelement und dem Erfassungsmittel im Normalbetrieb bzw. im Betrieb mit einem Leck,
- Fig. 3: eine Innenansicht eines Gehäuses einer Heizvorrichtung mit dem Erfassungsmittel,
- Fig. 4: eine schematische Darstellung des Schaltplans der Heizvorrichtung mit dem Erfassungsmittel, und
- Fig. 5: ein Ablaufdiagramm eines Verfahrens zum Detektieren von Verbrennungsgas eines Heizelements durch das Erfassungsmittel.

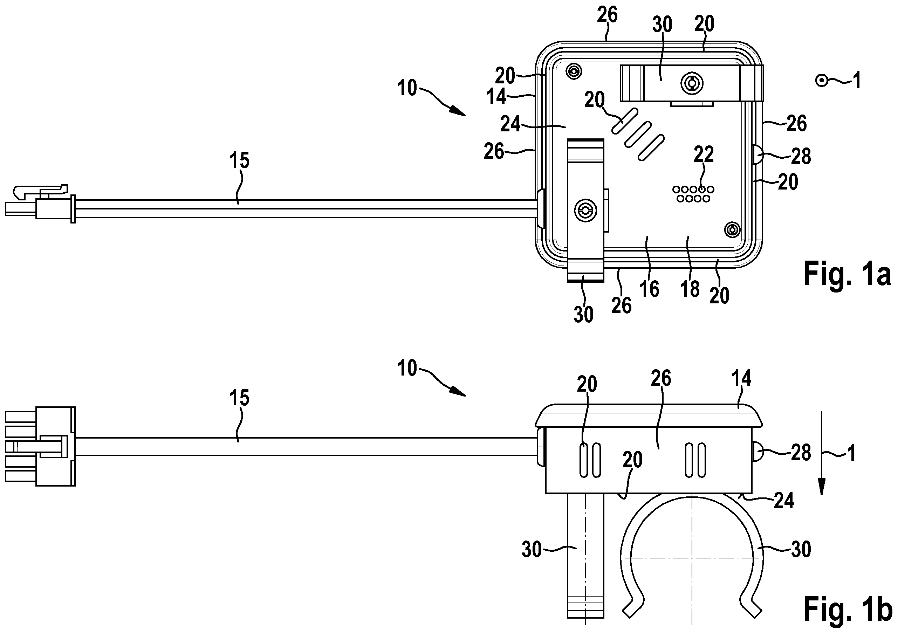

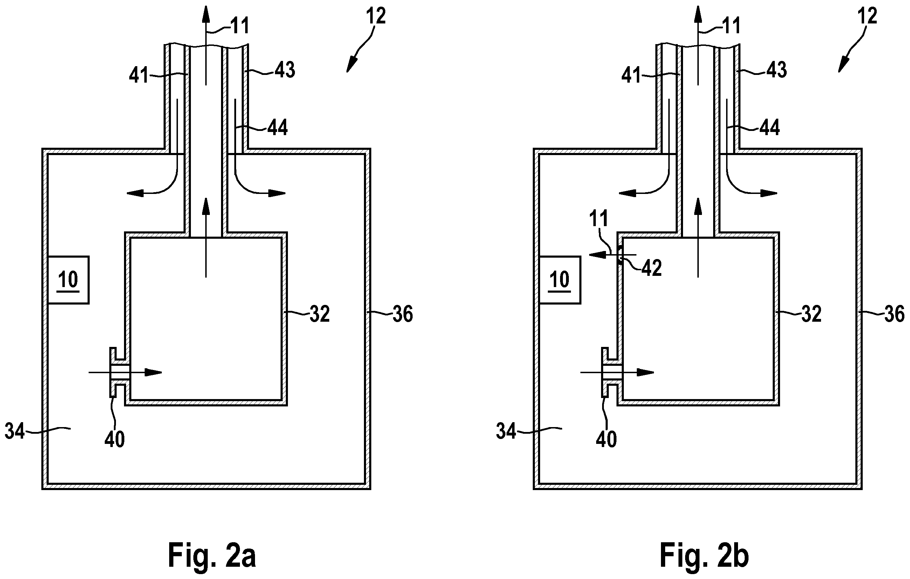

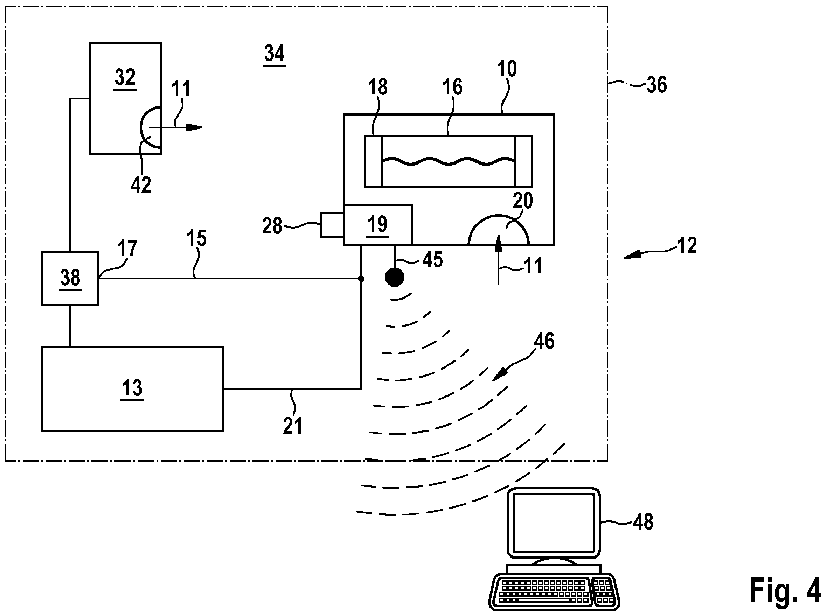

Figur 1 offenbart ein Erfassungsmittel 10 zum Detektieren von Verbrennungsgas 11 innerhalb einer Heizvorrichtung 12, wie sie in den Figuren 2 bis 4 dargestellt ist. Das Erfassungsmittel 10 wird durch eine Umhausung 14 gebildet, in der eine Spektroskopieeinheit 16 und einen Sensor 18 angeordnet sind. Die Spektroskopieeinheit 16 kann eine Infrarotspektroskopiemethode ausführen und beispielsweise eine Konzentration von CO2 als Verbrennungsgas 11 durch den Sensor 18 messen. Die Spektroskopieeinheit 16 und der Sensor 18 sind von der Umhausung 14 umgeben, wobei die Umhausung 14 wenigstens eine Öffnung 20 mit vorzugsweise in etwa 19 mm² aufweist, durch welches das Verbrennungsgas 11 in die Umhausung zum 14 eintreten kann, um von der Spektroskopieeinheit 16 spektroskopiert und durch den Sensor 18 gemessen zu werden. Ferner erstreckt sich von einer Seitenfläche 26 der Umhausung 14 ausgehend ein kabelförmiger Netzanschluss 15, welcher mittels einem Stecker an eine Spannungsversorgung 17 vorzugsweise innerhalb der Heizvorrichtung 12 angeschlossen werden kann.

Figur 1A zeigt eine Unteransicht des Erfassungsmittels 10, wobei die Öffnung 20 durch drei schlitzartige Öffnungen in der Unterseite 24 gebildet ist. Die schlitzartigen Öffnungen sind gleich lang und ungefähr diagonal zum quadratischen beziehungsweise rechteckigen Querschnitt der Umhausung 14 ausgerichtet.

Ferner sind Kontakte 22 für eine Datenübertragung auf der Unterseite 24 angeordnet. Die Kontakte 22 sind unmittelbar von außen zugänglich, sodass sie durch ein externes Gerät kontaktiert werden können. Über die Kontakte 22 können Daten ein- und ausgelesen werden. Beispielsweise werden Messdaten und Ereignisse der Heizvorrichtung 12 beziehungsweise des Erfassungsmittels 10 in einen Speicher einer Erfassungsmittelsteuerung 19 abgelegt, sodass diese Messdaten und die Daten zu den Ereignissen über die Kontakte 22 ausgelesen werden können. Weiter können Steuerungsbefehle über die Kontakte 22 an das Erfassungsmittel 10 übermittelt werden. Es sind insbesondere elf Kontaktpins in zwei Reihen positioniert. Dadurch ist die Öffnung 20 und die Kontakte 22 bezüglich der Erdanziehungsrichtung 1 auf einer nach unten gerichteten Seite der Umhausung 14 positioniert.

Auf der Unterseite 24 sind zwei Befestigungsmittel 30 angeordnet, die als Schellen ausgebildet sind, deren Vorzugsrichtungen um 90° zueinander versetzt sind, sodass die Schellen zwei Abschnitte eines Rohrs eingreifen können, die zueinander um 90° versetzt verlaufen. Die Schellen sind in von der Unterseite 24 wegweisender Weise geöffnet. Ferner sind die Schellen im Bereich des Rands der Unterseite 24 angeordnet, sodass zwischen den Schellen die Öffnung 20 und die Kontakte 22 positioniert sind.

An den Seitenflächen 26 sind weitere Löcher 20 ausgebildet, die durch bezüglich der Erdanziehungsrichtung 1 vertikal ausgerichtete Schlitze gebildet sind. Es können jeweils vier Schlitze angeordnet sein, die parallel zueinander ausgebildet sind. Dabei können zwei Schlitze an einem rechten und zwei Schlitze an einem linken Rand der Seitenfläche 24 ausgeformt werden. An einer Seitenfläche 26 ist eine Anzeige 28 in Form einer LED in etwa mittig angeordnet. Auf der zur Anzeige 28 gegenüberliegenden Seitenfläche 26 ist der Netzanschluss 15 angebracht. Die Anzeige 28 dient dazu einen Zustand der Heizvorrichtung 12 und/oder des Erfassungsmittels 10 anzuzeigen. Dabei kann die Anzeige 28 blinken oder durchgehend leuchten. Alternativ zu einer LED kann ein Display vorgesehen werden, welches in Form von Text oder Symbolen den Zustand der Heizvorrichtung 12 und/oder des Erfassungsmittels 10 anzeigt. Weiter alternativ ist eine akustische Ausgabe in Form einer Sprachausgabe oder eines Signaltons denkbar. Die Anzeige 28 ist zwischen den links und rechts auf der Seitenwand angeordneten Löchern 26 positioniert.

In Figur 1B ist eine Seitenansicht des Erfassungsmittels 10 dargestellt. Die Befestigungsmittel 30 weisen eine U-förmige Ausformung auf, die in Erdanziehungsrichtung 1 nach unten geöffnet sind. Die Umhausung 14 beinhaltet die Spektroskopieeinheit 16 und den Sensor 18 in einem schalenartigen Teil der durch einen Deckel abgeschlossen ist. Der Deckel ist auf der Oberseite auf dem schalenartigen Teil aufgesetzt, wobei die Oberseite der Unterseite 24 gegenüberliegend positioniert ist. Die Unterseite 24 bildet den Boden des schalenartigen Teils. Das Erfassungsmittel 10 wird in der in Figur 1B gezeigten Position in der Heizvorrichtung 12 eingebaut, sodass die Unterseite 24 mit der Öffnung 20 nach unten weist.

Figur 2A zeigt eine schematische Darstellung der Heizvorrichtung 12 mit einem Innenraum 34 im Normalbetrieb, welcher durch ein Gehäuse 36 gebildet wird. Das Gehäuse 36 bildet die äußere Hülle der Heizvorrichtung 12. In dem Innenraum 34 ist ein Heizelement 32 angeordnet, welches über ein Ansaugrohr 40 Luft 44 für eine Verbrennung in dem Heizelement 32 ansaugt. Durch die Verbrennung wird das Verbrennungsgas 11 erzeugt, welches wiederum durch ein Abgasrohr 41 aus dem Innenraum 34 befördert wird. Das Abgasrohr 41 ist konzentrisch zu einem Zuluftrohr 43, über welches frische Luft 44 in den Innenraum 43 gesaugt wird. Weiter ist in dem Innenraum 34 ein Erfassungsmittel 10 angeordnet, mit dem die Konzentration der Verbrennungsgase in dem Innenraum 34 erkannt werden kann. Verbrennungsgas kann CO2 sein und die Verbrennung kann eine Umsetzung von kohlenstoffbasierten Brennstoffen wie Erdgas und/oder Öl sein. Bei dieser Art von Verbrennung entsteht neben CO2 noch H2O.

In Figur 2B weist das Heizelement 32 ein Leck 42 auf, aus dem Verbrennungsgas 11 in den Innenraum 34 strömt. Durch das Leck 42 gelangt auch Feuchtigkeit durch das aus der Verbrennung stammende H2O in den Innenraum, sodass neben der Erhöhung der Konzentration an Verbrennungsgas 11 auch die Feuchtigkeit erhöht wird. Als 3. Größe kann die Temperatur im Innenraum erhöht werden, da das Verbrennungsgas 11 gewisse über dem Durchschnitt liegende Wärmemenge mit sich bringt. Die derartig mit CO2 und H2O angereicherte Luft 44 wird durch das Ansaugrohr 40 wiederum in das Heizelement 23 für die Verbrennung gesaugt. Dies kann zur Minderung der Effizienz der Verbrennungsführung. Um den Zustand der Heizvorrichtung 12 durch das Erfassungsmittel 10 sicher bestimmen zu können, kann neben der Spektroskopieeinheit 18 mit dem Sensor 16, ein Temperatursensor und/oder ein Feuchtigkeitssensor enthalten sein, mittels dem weitere Parameter die auf ein Leck 42 hinweisen, gefasst werden.

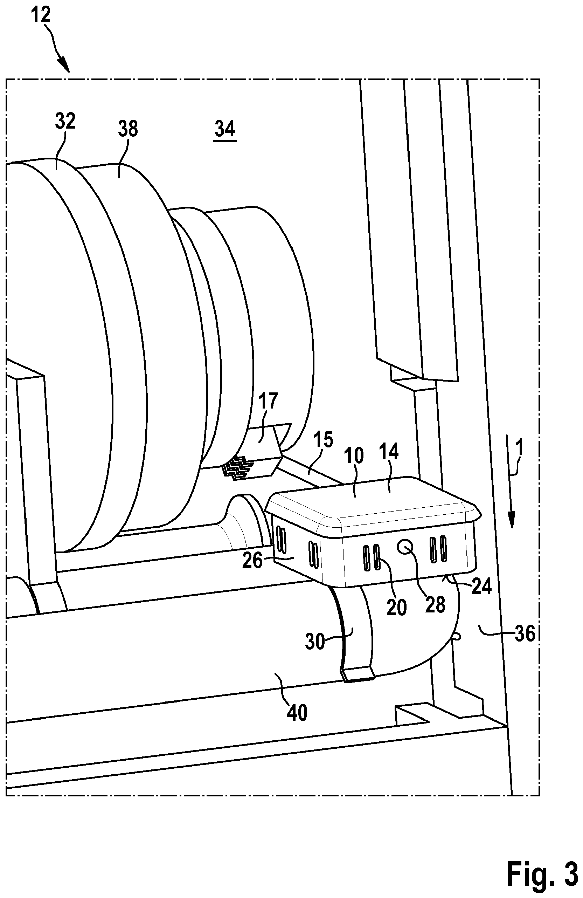

Figur 3 zeigt einen Ausschnitt eines durch ein Gehäuse 36 gebildeten Innenraums 34 einer Heizvorrichtung 12. In dem Innenraum ist ein Gebläse 38 angeordnet, welches Luft 44 ansaugt und über das Ansaugrohr 40 in das Heizelement 32 befördert. Das Ansaugrohr 40 ist in einem unteren Teil des Innenraums 34 positioniert und mäanderartig geformt. Auf eine Biegung des Ansaugrohr 40 ist das Erfassungsmittel 10 mit seinen Befestigungsmitteln 30 angeordnet. Dabei umgreifen die Schellen, welche die Befestigungsmittel 30 stellen, das Ansaugrohr 40. Dabei ist das Ansaugrohr 40 bezüglich der Erdanziehungsrichtung 1 unterhalb des Erfassungsmittels 10, sodass das Ansaugrohr 40 auf der Unterseite 24 eingeordnet ist. Die Anzeige 28 ist in Richtung einer Öffnung des Gehäuses 36 der Heizvorrichtung 12 ausgerichtet. Ferner wird der Netzanschluss 15 in eine Spannungsversorgung 17 des Gebläses 38 eingesteckt, wobei weitere Bauteile der Heizvorrichtung 12 zu dem Netzanschluss des Erfassungsmittels 10 parallelgeschaltet werden können.

Figur 3 zeigt einen schematischen Schaltplan einer Heizvorrichtung 12, welche ein Gehäuse aufweist, dass einen Innenraum 34 umfasst. In dem Innenraum 34 ist das Heizelement 32 angeordnet, welches ein Leck 42 aufweist, aus dem Verbrennungsgas 11 in den Innenraum 34 austritt. Das Gebläse 38 ist mit dem Heizelement 32 verbunden und wird durch eine Steuerung 13 der Heizvorrichtung 12 angesteuert. Das Erfassungsmittel 10 ist über den Netzanschluss 15. Gebläse 38 und optional mit einer Datenverbindung 21 mit der Steuerung 13 verbunden. Dabei umfasst das Erfassungsmittel 10 neben der Spektroskopieeinheit 16 und dem Sensor 18 eine Erfassungsmittelsteuerung 19, welche die Anzeige 28 ansteuert und den Sensor 18 auswertet. Bitte Erfassungsmittelsteuerung 19 ist eine Antenne 45 des Erfassungsmittels 10 verbunden, über die Daten durch ein Funksignal 46 ein und ausgelesen werden können. Tritt nun durch die Öffnung 20 eine Mischung aus Luft und Verbrennungsgas 11 in das Erfassungsmittel 10 ein, wird es von der Spektroskopieeinheit 16 spektroskopiert und vom Sensor 18 gemessen. Die Steuerung 19 wertet das Messsignal des Sensors 18 aus und kommuniziert sie über die Datenverbindung 21 an die Steuerung 13 und/oder über die Antenne 45 und das Funksignal 46 an einen externen Computer 48. Der externe Computer 48 kann ein Smartphone, ein Tablet, ein Laptop und/oder sonstiges externes Gerät sein auf dem insbesondere ein für das Erfassungsmittel 10 eingerichtetes Programm ausgeführt werden kann. Das Funksignal 46 kann eine Bluetoothverbindung, eine WLAN-Verbindung, eine UMTS-Verbindung, eine LTE-Verbindung und/oder eine sonstige Funkverbindung herstellen. Die Auswertung des Messsignals des Sensors 18 kann auch an die Anzeige 28 übermittelt werden, die einem Nutzer ein visuelles Signal über den Zustand der Heizvorrichtung 12 und/oder des Erfassungsmittels 10 mitteilt.

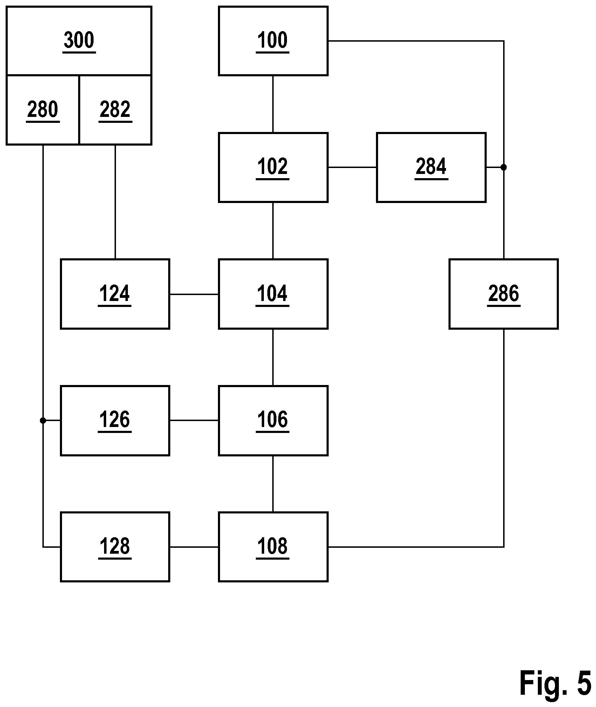

Figur 5 zeigt ein Ablaufdiagramm eines Verfahrens, welches von dem Erfassungsmittel 10 ausgeführt wird. Ein Start 100 des Verfahrens erfolgt beim Einschalten der Energieversorgung durch beispielsweise einstecken des Netzanschlusses 10 in die Spannungsversorgung 17 des Gebläses 38. Nach dem Start 100 prüft die Erfassungsmittelsteuerung 19 zuerst ob ein Fehlersignal und/oder Warnsignal vorliegt. Anschließend oder parallel wird eine Regelschleife eingesetzt, bei der eine Überprüfung 102 der Drehzahl des Gebläses 38 erfolgt. Dabei muss die Drehzahl des Gebläses 38 einen bestimmten in der Erfassungsmittelsteuerung 19 hinterlegten Wert aufweisen. Als die Drehzahl des Gebläses 38 diesen Wert nicht auf, so wird ein Signal an eine Anzeige 28 gesendet, und die Anzeige 28 wird in einen blinkenden Anzeigezustand 284 versetzt, bei dem die Anzeige vorzugsweise 0,1 s leuchtet und 0,9 s aus ist. Insbesondere kann ein Signal an die Steuerung 13 der Heizvorrichtung 12 übermittelt werden. Ferner kann ein Signal an einen externen Computer 48 gesendet werden. An Servicetechniker oder ein Nutzer kann über die Steuerung 13 der Heizvorrichtung 12, die Anzeige 28 des Erfassungsmittels 10 und/oder über den externen Computer 48 über die zu geringe Drehzahl des Gebläses 38 informiert werden. Wenn die Bedingung der Drehzahl nicht erfüllt ist, prüft die Erfassungsmittelsteuerung 19 nach einer vorher gesetzten Zeitdauer für das Wiederholen der Prüfung die Drehzahl. Die Zeitdauer kann beispielsweise 15 Hz betragen, sodass die Prüfung mit 15 Hz wiederholt wird. Dies ist die Frequenz der Wiederholung der Regelschleife, wenn die Bedingung der Drehzahl des Gebläses 38 nicht erfüllt ist.

Falls die Drehzahl dem erwarteten Wert entspricht wird eine Überprüfung 104 des Sensors 18 durchgeführt. Dabei wird durch die Erfassungsmittelsteuerung 19 das Signal des Sensors 18 ausgewertet und eine Konzentration CO2_sensor bestimmt und mit einem unteren Schwellenwert CO2_min verglichen. Ist die Konzentration CO2_sensor kleiner oder gleich dem unteren Schwellenwert CO2_min noch, dann wertet die Erfassungsmittelsteuerung 19 dies als zu zählendes Ereignis. Somit wird jedes Mal ein Zählwert durch einen Zähler 124 in der Erfassungsmittelsteuerung 19 erhöht, wenn die Bedingung
CO2_sensor <= CO2_min
erfüllt wird. Der untere Schwellenwert CO2_min der Konzentration von CO2 kann zwischen 0 und 400 ppm in der Luft des Innenraums 34 der Heizvorrichtung 12 liegen. Wird eine bestimmte Häufigkeit innerhalb einer bestimmten Zeitspanne durch den Zielwert erreicht, dann wird ein Fehlersignal ausgegeben, welches die Anzeige 28 in einen blinkenden Anzeigezustand 282 versetzt und beispielsweise das Gebläse 38 in einen gestoppten Zustand 300 versetzen, indem es die den Motor des Gebläses 38 antreibende Transistorbrücke spannungsfrei schaltet. Alternativ oder ergänzend kann die Steuerung 13 der Heizvorrichtung blockiert werden und/oder die Verbrennung innerhalb des Heizelements 12 verringert und/oder konstant und/oder erhöht werden. Der Anzeigezustand 282 kann sich durch ein blinken mit einer 0,1 s langen An- und Ausphase auszeichnen. Die bestimmte Häufigkeit kann einen Wert von 20 und die bestimmte Zeitdauer 30 s betragen.

Wird der untere Schwellenwert CO2_min nicht erreicht wird ein oberer Schwellenwert CO2_max bei einer Überprüfung 106 anhand der Bedingungen
CO2_sensor > CO2_max
kontrolliert. Der obere Schwellenwert CO2_max für die Konzentration von CO2 in der Luft des Innenraums 23 kann zwischen 2000 und 10000 ppm. Wird diese Bedingung erfüllt, erhöht ein Zähler 126 einen Zählwert. Erreichte Zählwert einen bestimmten Betrag von beispielsweise 40 innerhalb einer bestimmten Zeit oder über die gesamte Zeitdauer des Betriebs der Heizvorrichtung 12, dann wird ein Warnsignal an die Anzeige 28 versendet, dass die Anzeige in einen durchgehend leuchtenden Anzeigenzustand 280 versetzt. Ferner kann das Gebläse 38 in einen gestoppten Zustand 300 durch das Warnsignal versetzen werden, indem es die den Motor des Gebläses 38 antreibende Transistorbrücke spannungsfrei schaltet. Alternativ oder ergänzend kann die Steuerung 13 der Heizvorrichtung blockiert werden und/oder die Verbrennung innerhalb des Heizelements 12 verringert und/oder konstant und/oder erhöht werden.

Das Fehlersignal und das Warnsignal können innerhalb des Erfassungsmittels 10 verarbeitet werden und/oder über eine Datenverbindung 21 die Steuerung 13 der Heizvorrichtung 12 gesendet werden. Innerhalb des Erfassungsmittels 10 kann das Fehlersignal und das Warnsignal durch eine Treiberschaltung für die Anzeige 28, die Teil der Erfassungsmittelsteuerung 19 ist, aufbereitet werden. Das Fehlersignal und/oder das Warnsignal können auch an ein externes Gerät wie einem Computer, einem Smart Phone und/oder ein Tablet übermittelt werden.

Wenn das Fehlersignal und/oder das Warnsignal ausgegeben wurden und die Heizvorrichtung 12 und/oder das Erfassungsmittel 10 in einen entsprechenden Zustand, wie zum Beispiel einen Stoppzustand 300, versetzt wurden, kann mittels eines Fortführbefehls die Heizvorrichtung 12 und/oder das Erfassungsmittel 10 dennoch weiter betrieben werden. Dazu wird der Fortführbefehl in die Erfassungsmittelsteuerung 19 eingegeben. Die Häufigkeit des Fortführbefehls wird durch einen Zähler 128 innerhalb einer bestimmten Zeitdauer von zum Beispiel 48 h gezählt. Überschreitet die Häufigkeit des Fortführbefehls einen bestimmten Zählwert wie zum Beispiel 40 Fortführbefehle, dann wird das Warnsignal ausgegeben, und das Erfassungsmittel 10 und/oder die Heizvorrichtung 12 in den gleichen Zustand versetzt als wäre der obere Schwellenwert CO2_max der Konzentration des Verbrennungsgases 11 überschritten worden.

Falls die Häufigkeit des Fortführbefehls den bestimmten Zählwert innerhalb der bestimmten Zeitdauer nicht erreicht, läuft das Verfahren im Normalbetrieb weiter, was durch einen Anzeigenzustand 286 signalisiert wird, bei dem beispielsweise ein blinken der Anzeige mit 1 Hz zu verzeichnen ist. Im Normalbetrieb läuft die Regelschleife im normalen Zustand ab und mit der anfänglich festgelegten Frequenz von 15 Hz wiederholt werden. Dabei werden sämtliche Prüfungen der oberen und unteren Schwellenwerten Konzentration des Verbrennungsgases 11 als auch Drehzahl des Gebläses 38 wiederholt.

### Bezugszeichenliste

- 1: Erdanziehungsrichtung
- 10: Erfassungsmittel
- 11: Verbrennungsgas
- 12: Heizvorrichtung
- 13: Steuerung Heizvorrichtung
- 14: Umhausung
- 15: Netzanschluss
- 16: Spektroskopieeinheit
- 17: Spannungsversorgung
- 18: Sensor
- 19: Erfassungsmittelsteuerung
- 20: Öffnungen
- 22: Kontakte
- 24: Unterseite
- 26: Seitenfläche
- 28: Anzeige
- 30: Befestigungsmittel
- 32: Heizelement
- 34: Innenraum
- 36: Gehäuse
- 38: Gebläse
- 40: Ansaugrohr
- 42: Leck
- 44: Luft
- 46: Funksignal
- 48: externer Computer
- 100: Start des Verfahrens
- 102: Prüfung Drehzahl des Gebläses
- 104: Prüfung unterer Schwellenwert der Konzentration
- 106: Prüfung oberer Schwellenwert der Konzentration
- 108: Häufigkeit des Fortführbefehls innerhalb einer bestimmten Zeitspanne
- 124: Häufigkeit Erreichen/Unterschreiten des unteren Schwellenwerts
- 126: Häufigkeit Erreichen/Unterschreiten des oberen Schwellenwerts
- 128: Häufigkeit der Zeitspanne des Fortführbefehls
- 280: Anzeigenzustand für Stopp durch Überschreiten des Schwellenwerts
- 282: Anzeigenzustand für Stopp durch Unterschreiten des Schwellenwerts
- 284: Anzeigenzustand für zu niedrige Drehzahl des Gebläses
- 286: Anzeigenzustand für Normalbetrieb
- 300: Stopp der Heizvorrichtung

## Patentansprüche

1. Verfahren zum Detektieren von Verbrennungsgas (11) eines Heizelements (32) durch ein Erfassungsmittel (10) in einem durch ein Gehäuse (36) einer Heizvorrichtung (12) gebildeten Innenraum (34), wobei das Erkennen durch eine spektroskopische Messmethode mittels einer Spektroskopieeinheit (16) und einen Sensor (18) innerhalb einer Umhausung (14) des Erfassungsmittels (10) erfolgt, sodass bei Überschreiten eines oberen Schwellenwerts der Konzentration des Verbrennungsgases (11) im Innenraum (34) durch eine Erfassungsmittelsteuerung (19) ein Warnsignal von dem Erfassungsmittel (10) ausgegeben wird, wobei dass das Erfassungsmittel (10) die Konzentration des Verbrennungsgases (11) in der Luft des Innenraums (34) detektiert, wobei der obere Schwellenwert der Konzentration in etwa zwischen 2000 und 10000 ppm und insbesondere bei 7000 ppm liegt, wobei der obere Schwellenwert vorzugsweise in einem Speicher in der Erfassungsmittelsteuerung (19) hinterlegt ist, wobei vorzugsweise vor dem Messen der Konzentration des Verbrennungsgases (11) durch die Erfassungsmittelsteuerung (19) geprüft wird, ob der Sensor (18) eine Konzentration erkennt, die unter oder gleich einem bestimmten unteren Schwellenwert liegt, wobei der untere Schwellenwert insbesondere in etwa zwischen 0 und 400 ppm liegt, und wenn die Konzentration unter dem unteren Schwellenwert liegt, wird von der Erfassungsmittelsteuerung (19) ein Fehlersignal ausgegeben.

2. Verfahren nach Anspruch 1, wobei die bestimmte Konzentration eine bestimmte Zeitspanne lang durch das Erfassungsmittel (10) erkannt wird.

3. Verfahren nach einem der vorhergehenden Ansprüche, wobei eine Häufigkeit der Überschreitung des oberen Schwellenwerts durch die Erfassungsmittelsteuerung (19) insbesondere während einer bestimmten Zeitspanne gezählt wird, und das Warnsignal erst ausgegeben wird, wenn die Häufigkeit einen bestimmten Häufigkeitswert überschreitet.

4. Verfahren nach einem der vorhergehenden Ansprüche, wobei eine Häufigkeit der Unterschreitung des unteren Schwellenwerts durch die Erfassungsmittelsteuerung (19) insbesondre während einer bestimmten Zeitspanne gezählt wird, und das Fehlersignal erst ausgegeben wird, wenn die Häufigkeit einen bestimmten Häufigkeitswert überschreitet.

5. Verfahren nach einem der vorhergehenden Ansprüche, wobei das Erfassungsmittel (10) das Warnsignal und/oder das Fehlersignal an eine Steuerung (13) der Heizvorrichtung (12) und/oder an eine Anzeige (28) des Erfassungsmittels (10) abgibt.

6. Verfahren nach einem der vorhergehenden Ansprüche, wobei die Steuerung (13) die Heizvorrichtung (12) zu einer Verringerung der Konzentration des Verbrennungsgases veranlasst.

7. Verfahren nach einem der vorhergehenden Ansprüche, wobei das Warnsignal ein Ausschalten der Heizvorrichtung (12) auslöst, sodass die Verbrennung erlischt und/oder ein Blockieren der Steuerung (13) der Heizvorrichtung (12) auslöst und/oder eine Verringerung der Verbrennung innerhalb des Heizelements (32) bewirkt.

8. Verfahren nach einem der vorhergehenden Ansprüche, wobei die Heizvorrichtung (12) nach dem Ausgeben des Warnsignals und/oder des Fehlersignals durch einen Fortführbefehl weiterbetreibar ist, wobei die Erfassungsmittelsteuerung (19) die Häufigkeit der Fortführbefehle insbesondre während einer bestimmten Zeitspanne zählt, und bei Überschreiten einer bestimmten Häufigkeit, die Heizvorrichtung (12) ausgeschaltet wird, und/oder die Steuerung (13) der Heizvorrichtung (12) blockiert wird und/oder die Verbrennung verringert wird und/oder der Fortführbefehl blockiert wird.

9. Verfahren nach einem der vorhergehenden Ansprüche, wobei eine Anzeige (28), die Warnsignal und/oder das Fehlersignal empfängt, an der Umhausung (14) angeordnet ist, wobei die Anzeige (28) wenigstens eine LED ist, die je nach Messergebnis des Sensors () blinkt oder ausgeschaltet ist oder durchgehend leuchtet, wobei die LED insbesondere in unterschiedlichen Farben leuchten kann.

10. Verfahren nach einem der vorhergehenden Ansprüche, wobei die Erfassungsmittelsteuerung (19) über eine Antenne Funksignale () an ein bezüglich des Erfassungsmittels (10) externes Gerät wie einem Computer, einem Smart Phone und/oder ein Tablet sendet, und einen Zustand der Heizvorrichtung (12) und/oder eine Konzentration des Verbrennungsgases (11) und/oder ausgegebene Signale und/oder Häufigkeiten anzeigt.

11. Verfahren nach einem der vorhergehenden Ansprüche, wobei über die Antenne Funksignale empfangbar sind, sodass durch ein externes Gerät wie einem Computer, einem Smart Phone und/oder ein Tablet die Erfassungseinheit steuerbar ist.

12. Verfahren nach einem der vorhergehenden Ansprüche, wobei wenigstens ein Signal eines Feuchtigkeitssensors, Drucksensor, Drehzahlmessers eines Gebläses (38) der Heizvorrichtung (12) und/oder eines Temperatursensors von der Erfassungsmittelsteuerung (19) herangezogen wird, um den oberen Schwellenwert der Konzentration entsprechend anzupassen.

13. Verfahren nach einem der vorhergehenden Ansprüche, wobei eine Drehzahl eines Gebläses (38) abgeprüft wird, und wenn die Drehzahl des Gebläses (38) niedriger als ein Sollwert ist, wird eine Anzeige (28) wie eine LED des Erfassungsmittels (10) insbesondere in einen blinkenden Zustand eingeschaltet.

## Claims

1. Method for detecting combustion gas (11) of a heating element (32) by way of a detection means (10) in an interior (34) formed by a housing (36) of a heating device (12), wherein the detection is carried out by way of a spectroscopic measurement method using a spectroscopy unit (16) and a sensor (18) within an enclosure (14) of the detection means (10), with the result that, if an upper threshold value of the concentration of the combustion gas (11) in the interior (34) is exceeded, a detection means controller (19) outputs a warning signal from the detection means (10), wherein the detection means (10) detects the concentration of the combustion gas (11) in the air of the interior (34), wherein the upper threshold value of the concentration is approximately between 2000 and 10,000 ppm and is in particular 7000 ppm, wherein the upper threshold value is preferably stored in a memory in the detection means controller (19), wherein, before measuring the concentration of the combustion gas (11), the detection means controller (19) preferably checks whether the sensor (18) detects a concentration that is below or equal to a specific lower threshold value, wherein the lower threshold value is in particular approximately between 0 and 400 ppm, and, if the concentration is below the lower threshold value, the detection means controller (19) outputs an error signal.

2. Method according to Claim 1, wherein the specific concentration is detected by the detection means (10) for a specific period of time.

3. Method according to one of the preceding claims, wherein a frequency with which the upper threshold value is exceeded is counted by the detection means controller (19), in particular during a specific period of time, and the warning signal is output only if the frequency exceeds a specific frequency value.

4. Method according to one of the preceding claims, wherein a frequency with which the lower threshold value is undershot, is counted by the detection means controller (19), in particular during a specific period of time, and the error signal is output only if the frequency exceeds a specific frequency value.

5. Method according to one of the preceding claims, wherein the detection means (10) emits the warning signal and/or the error signal to a controller (13) of the heating device (12) and/or to a display (28) of the detection means (10).

6. Method according to one of the preceding claims, wherein the controller (13) causes the heating device (12) to reduce the concentration of the combustion gas.

7. Method according to one of the preceding claims, wherein the warning signal triggers a switch-off of the heating device (12), with the result that the combustion is extinguished, and/or triggers blocking of the controller (13) of the heating device (12), and/or causes a reduction in the combustion inside the heating element (32) .

8. Method according to one of the preceding claims, wherein, after the warning signal and/or the error signal has/have been output, the heating device (12) can continue to be operated by way of a continuation command, wherein the detection means controller (19) counts the frequency of the continuation commands, in particular during a specific period of time, and, if a specific frequency is exceeded, the heating device (12) is switched off and/or the controller (13) of the heating device (12) is blocked and/or the combustion is reduced and/or the continuation command is blocked.

9. Method according to one of the preceding claims, wherein a display (28), which receives the warning signal and/or the error signal, is arranged on the enclosure (14), wherein the display (28) is at least one LED which, depending on the measurement result from the sensor (), flashes or is switched off or continuously emits light, wherein the LED can emit light of different colours, in particular.

10. Method according to one of the preceding claims, wherein the detection means controller (19) transmits radio signals () to a device outside the detection means (10), such as a computer, a smartphone and/or a tablet, via an antenna and displays a state of the heating device (12) and/or a concentration of the combustion gas (11) and/or output signals and/or frequencies.

11. Method according to one of the preceding claims, wherein radio signals can be received via the antenna, with the result that the detection unit can be controlled by an external device such as a computer, a smartphone and/or a tablet.

12. Method according to one of the preceding claims, wherein at least one signal from a humidity sensor, a pressure sensor, a tachometer of a fan (38) of the heating device (12) and/or a temperature sensor is used by the detection means controller (19) to accordingly adapt the upper threshold value of the concentration.

13. Method according to one of the preceding claims, wherein a speed of a fan (38) is checked and, if the speed of the fan (38) is lower than a target value, a display (28), such as an LED, of the detection means (10) is switched on in a flashing state, in particular.

## Revendications

1. Procédé de détection de gaz de combustion (11) d'un élément chauffant (32) par un moyen de détection (10) dans un espace intérieur (34) formé par un boîtier (36) d'un dispositif de chauffage (12), la reconnaissance étant effectuée par une méthode de mesure spectroscopique au moyen d'une unité de spectroscopie (16) et d'un capteur (18) à l'intérieur d'une enceinte (14) du moyen de détection (10), de sorte qu'en cas de dépassement d'une valeur de seuil haute de la concentration du gaz de combustion (11) dans l'espace intérieur (34), un signal d'avertissement est émis par le moyen de détection (10) par le biais d'une commande de moyen de détection (19), le moyen de détection (10) détectant la concentration du gaz de combustion (11) dans l'air de l'espace intérieur (34), la valeur de seuil haute de la concentration étant comprise approximativement entre 2 000 et 10 000 ppm et étant notamment de l'ordre de 7 000 ppm, la valeur de seuil haute étant de préférence enregistrée dans une mémoire dans la commande de moyen de détection (19), un contrôle étant de préférence effectué par la commande de moyen de détection (19) avant la mesure de la concentration du gaz de combustion (11) afin de vérifier si le capteur (18) reconnaît une concentration qui est inférieure ou égale à une valeur de seuil basse déterminée, la valeur de seuil basse étant notamment comprise approximativement entre 0 et 400 ppm, et si la concentration est inférieure à la valeur de seuil basse, un signal d'erreur étant délivré par la commande de moyen de détection (19).

2. Procédé selon la revendication 1, la concentration déterminée étant reconnue pendant une période déterminée par le moyen de détection (10).

3. Procédé selon l'une des revendications précédentes, une fréquence de dépassement du seuil haut étant comptée par la commande de moyen de détection (19), notamment pendant une période déterminée, et le signal d'avertissement n'étant délivré que lorsque la fréquence dépasse une valeur de fréquence déterminée.

4. Procédé selon l'une des revendications précédentes, une fréquence de franchissement vers le bas du seuil bas étant comptée par la commande de moyen de détection (19), notamment pendant une période déterminée, et le signal d'erreur n'étant délivré que lorsque la fréquence dépasse une valeur de fréquence déterminée.

5. Procédé selon l'une des revendications précédentes, le moyen de détection (10) délivrant le signal d'avertissement et/ou le signal d'erreur à une commande (13) du dispositif de chauffage (12) et/ou à un indicateur (28) du moyen de détection (10).

6. Procédé selon l'une des revendications précédentes, la commande (13) amenant le dispositif de chauffage (12) à une réduction de la concentration du gaz de combustion.

7. Procédé selon l'une des revendications précédentes, le signal d'avertissement déclenchant une mise à l'arrêt du dispositif de chauffage (12) de sorte que la combustion s'éteint et/ou déclenche un blocage de la commande (13) du dispositif de chauffage (12) et/ou provoque une diminution de la combustion à l'intérieur de l'élément chauffant (32).

8. Procédé selon l'une des revendications précédentes, le dispositif de chauffage (12) pouvant continuer à fonctionner par une instruction de poursuite après la délivrance du signal d'avertissement et/ou du signal d'erreur, la commande de moyen de détection (19) comptant la fréquence des instructions de poursuite, notamment pendant une période déterminée et, en cas de dépassement d'une fréquence déterminée, le dispositif de chauffage (12) est mis à l'arrêt et/ou la commande (13) du dispositif de chauffage (12) est bloquée et/ou la combustion est réduite et/ou l'instruction de poursuite est bloquée.

9. Procédé selon l'une des revendications précédentes, un indicateur (28), qui reçoit le signal d'avertissement et/ou le signal d'erreur, étant disposé sur l'enceinte (14), l'indicateur (28) étant au moins une LED qui, selon le résultat de mesure du capteur (), clignote ou est éteinte ou est allumée en continu, la LED pouvant notamment s'allumer dans différentes couleurs.

10. Procédé selon l'une des revendications précédentes, la commande de moyen de détection (19) émettant des signaux radio () par le biais d'une antenne vers un appareil externe au moyen de détection (10), tel qu'un ordinateur, un Smartphone et/ou une tablette, et indiquant un état du dispositif de chauffage (12) et/ou une concentration de gaz de combustion (11) et/ou des signaux délivrés et/ou des fréquences.

11. Procédé selon l'une des revendications précédentes, des signaux radio pouvant être reçus par le biais de l'antenne, de sorte que l'unité de détection peut être commandée par un appareil externe tel qu'un ordinateur, un Smartphone et/ou une tablette.

12. Procédé selon l'une des revendications précédentes, au moins un signal d'un capteur d'humidité, d'un capteur de pression, d'un capteur de vitesse de rotation d'un ventilateur (38) du dispositif de chauffage (12) et/ou d'un capteur de température étant utilisé par la commande de moyen de détection (19) afin d'ajuster en conséquence la valeur de seuil haute de la concentration.

13. Procédé selon l'une des revendications précédentes, une vitesse de rotation d'un ventilateur (38) étant vérifiée et, si la vitesse de rotation du ventilateur (38) est inférieure à une valeur de consigne, un indicateur (28) tel qu'une LED du moyen de détection (10) étant allumé, notamment dans un état clignotant.
